# EUROPEAN PATENT APPLICATION

(11) **EP 2 959 935 A1**
(43) Date of publication of application: **30.12.2015**
(21) Application number: 14754438.1
(22) Date of filing: 06.02.2014
(51) Int. Cl.: A61M 39/02

(54) **VALVE FOR PREVENTING THE WASTE OF BLOOD**

(30) Priority: 19.02.2013 ES 201330215
(71) Applicant: FUNDACIÓN PARA LA INVESTIGACIÓN BIOMÉDICA DEL HOSPITAL 12 DE OCTUBRE, 28041 Madrid (ES)
(72) Inventor: VILLALTA GARCIA, Pedro, 28916 Madrid (ES)
(74) Representative: Lahidalga de Careaga, Jose Luis
(86) International application number: PCT/ES2014/070087
(87) International publication number: WO 2014/128323

(57) **Abstract**

An anti-blood-loss valve based on a valve body (6) of flexible, soft material, divided into two parts by an open/close valve (3) on each side of which two valves are seen, one for extracting blood for analysis (4) and the other for blood for reinfusion (5). The valve body (6) also has an input with a connector to the catheter (1) and, opposite, another connector to the serum and/or reinfusion system (2).

## Description

### OBJECT OF THE INVENTION

The invention proposed refers to an anti-blood-loss valve fitted in any intravenous tubing using a catheter so that, in addition to administering serotherapy, blood can be extracted for analysis in better conditions than at present.

### THE FIELD OF THE INVENTION

The field of the invention is that of manufacturers of both throwaway sanitary items and plastic products.

### PRIOR ART

There are some forerunners, of valves installed in a catheter for serotherapy, but none is known which is able to perform the two functions, to administer the serotherapy and extract blood without discarding any.

The advantage of the device proposed in the invention is that it improves the procedure for extracting blood so that when a patient needs an analysis, to avoid a direct puncture, the catheter installed to extract the blood for analysis can be employed using one of the tubes available.

It must be remembered that the serotherapy treatment flowing through the catheter includes ions, glucose and medication so that if it is necessary to take blood from this catheter to avoid giving the patient an injection, a certain amount of blood must be discarded so as not to alter the analytical results of the blood extracted, it is thrown away and cannot be recovered for the patient.

The invention put forward is able to secure a point of extraction, each time creating a closed system which avoids infection, complying with the "bacteriemia zero" standard, and the blood formerly discarded in order to extract blood for analysis is reinfused to the patient, who does not therefore suffer iatrogenic anaemia.

The inventor is unaware of any predecessor incorporating the arrangements of this invention, or the benefits of such arrangement.

### DESCRIPTION OF THE INVENTION

The invention proposed refers to an anti-blood-loss valve fitted to the catheter of a patient as part of any intravenous tubing using a catheter so that, in addition to administering a serotherapy treatment, blood can be extracted for analysis in better conditions that at present, and comprising a valve body (6) of flexible, soft material, divided into two parts by an open/close valve on each side of which two valves are seen, one for extracting blood for analysis and the other for blood for reinfusion.

The valve body also has an input with a connector to the catheter and, opposite, another connector to the serum and/or reinfusion system.

The procedure for extracting blood with the system functions as follows.

The fluid and blood is aspirated through a conventional syringe using the blood extraction valve and, without removing the syringe, the close/open valve is shut and another syringe is use to extract the blood from the patient though the blood extraction valve.

Once the blood has been extracted, the fluid and blood taken in the first place are reinjected in the valve for the extraction of the blood which is reinfused.

### DESCRIPTION OF THE DRAWINGS

For a better understanding of the invention, a page of plans is attached, showing the following:

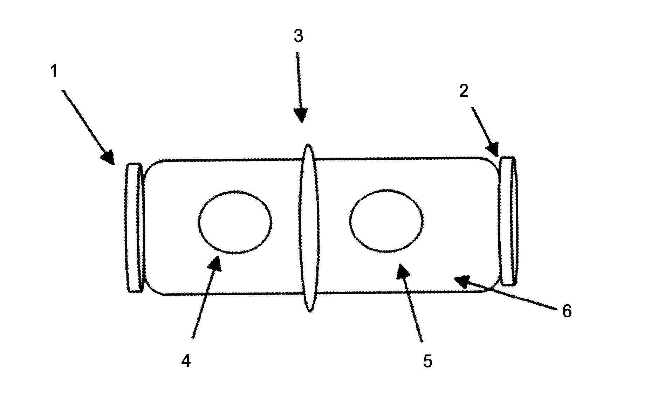

FIGURE 1.- A diagrammatic cross-section view of the valve.

And in that figure, identical elements are denoted with a single reference, among them the following:
(1).- connector to the catheter
(2).- connector to the serum supply
(3).- valve close/open device
(4).- valve for extraction of blood for analysis
(5).- valve for extraction of blood for reinfusion
(6).- valve body

### PREFERENTIAL EMBODIMENTS OF THE INVENTION

The invention proposed refers to an anti-blood-loss valve fitted to the catheter of a patient as part of any intravenous tubing using a catheter so that, in addition to administering a serotherapy treatment, blood can be extracted for analysis in better conditions that at present, and comprising a valve body (6) of flexible, soft material, divided into two parts by an open/close valve (3) on each side of which two valves are seen, one for extracting blood for analysis (4) and the other for blood for reinfusion (5).

The valve body (6) also has an input with a connector to the catheter (1) and, opposite, another connector to the serum and/or reinfusion system (2).

The procedure for extracting blood with the system functions as follows.

The fluid and blood is aspirated through a conventional syringe via the blood extraction valve (5) and, without removing the syringe, the close/open valve (3) is shut and another syringe is used to extract the blood from the patient though the blood extraction valve (4).

Once the blood has been extracted, the fluid and blood taken in the first place are injected once more in the valve for the extraction of the blood which is reinfused (5).

Having sufficiently described the nature of the invention and its practical implementation, it must be recorded that the specifications indicated above and represented in the attached drawings may be modified in detail provided that this does not alter their fundamental principles established in the previous paragraphs and summarised in the following claims.

## Claims

1. An anti-blood-loss valve, fitted to the catheter of a patient as part of any intravenous tubing using a catheter, **characterised by** being based on a valve body (6) of flexible, soft material, divided into two parts by an open/close valve (3) on each side of which two valves are seen, one for extracting blood for analysis (4) and the other for blood for reinfusion (5).
The valve body (6) also has an input with a connector to the catheter (1) and, opposite, another connector to the serum and/or reinfusion system (2).
